# FASCICULE DE BREVET EUROPEEN

(11) **EP 3 038 593 B1**
(45) Date de publication et mention de la délivrance du brevet: **27.01.2021**
(21) Numéro de dépôt: 14758135.9
(22) Date de dépôt: 29.08.2014
(51) Int. Cl.: A61K 8/36, A61Q 5/00, A61Q 5/02, A61K 8/67

(54) **COMPOSITION À BASE D'ACIDE GRAS POLYINSATURÉ ET DE VITAMINE D DESTINÉE À UNE ADMINISTRATION PAR VOIE ORALE POUR AMÉLIORER LA QUALITÉ DE LA CHEVELURE**
ORAL VERABREICHBARE ZUSAMMENSETZUNG MIT MEHRFACH UNGESÄTTIGTEN FETTSÄUREN UND VITAMIN D ZUR VERBESSERUNG DER HAARQUALITÄT.
ORAL COMPOSITION FOR IMPROVING HAIR QUALITY COMPRISING POLYINSATURATED FATTY ACIDS AND VITAMIN D

(30) Priorité: 30.08.2013 FR 1358349
(43) Date de publication de la demande: 06.07.2016
(73) Titulaire: NUTRICOS Technologies, 92117 Clichy Cedex (FR)
(72) Inventeur: BRU, Carole, F-92400 Courbevoie (FR); MAHE, Yann, F-91700 Sainte Geneviève des Bois (FR); PICCARDI, Nathalie, F-21310 Arceau (FR)
(74) Mandataire: Lavoix
(86) Numéro de dépôt international: PCT/EP2014/068450
(87) Numéro de publication internationale: WO 2015/028648

(56) Documents cités:
- EP-A1- 1 932 509
- FR-A- 1 601 008
- US-A1- 2005 175 565
- US-B1- 8 168 611
- Life Pharmacy: "Nutra-Life Omega 3 Fish Oil 1500mg Plus Vitamin D 180s", , 8 février 2013 (2013-02-08), XP002726524, Extrait de l'Internet: URL:http://wayback.archive.org/web/2013020 8050721/http://www.lifepharmacy.co.nz/p/Nu tra-Life-Omega-3-Fish-Oil-1500mg-Plus-Vita min-D-180s?i=128647 [extrait le 2014-06-30]
- Swanson Health Products: "Omega Swirl Fish Oil with Vitamin D Mango Peach", , 30 novembre 2012 (2012-11-30), XP002726525, Extrait de l'Internet: URL:http://www.swansonvitamins.com/barlean s-omega-swirl-fish-oil-vitamin-d-mango-pea ch-16-oz-liquid [extrait le 2014-06-26]
- DEMCHENKO DMITRY V ET AL: "Validated HPTLC Method for Quantification of Vitamin D-3 in Fish Oil", JPC. JOURNAL OF PLANAR CHROMATOGRAPHY, MODERN TLC, HUETHIG, HEIDELBERG, DE, vol. 24, no. 6, 1 décembre 2011 (2011-12-01), pages 487-490, XP009171431, ISSN: 0933-4173, DOI: 10.1556/JPC.24.2011.6.6
- "Skin, Hair & Nails Supplement", GNPD; MINTEL, November 2008 (2008-11), XP002699673, [retrieved on 2008-11-01]
- DATABASE GNPD [Online] MINTEL; November 2008 (2008-11), "Dietary Supplement with DHA Omega-3", Database accession no. 999084
- DATABASE GNPD [Online] MINTEL; September 2012 (2012-09), "Eye Plus Supplement Eye Sight+Protection", Database accession no. 1864261
- DATABASE GNPD [Online] MINTEL; July 2010 (2010-07), "Hair-Active Capsules", Database accession no. 1357230
- DATABASE GNPD [Online] MINTEL; July 2012 (2012-07), "Pure Cod Liver Oil", Database accession no. 1837427
- MonsieurCalvitie: "Vitamine D et perte de cheveux : des études", , 2012, Retrieved from the Internet: URL:https://monsieurcalvitie.wordpress.com /traitements-actuels/vitamine-d-et-perte-d e-cheveux-des-etudes/ [retrieved on 2018-06-20]

## Description

### Domaine de l'invention

La demande décrit l'utilisation cosmétique par voie orale d'une association d'actifs à base d'acide gras polyinsaturé et de vitamine D pour améliorer la qualité de la chevelure.

### Etat de la technique

Les cheveux sont produits dans des follicules pileux formés de gaines épithéliales d'origine épidermique et d'un bulbe pileux contenant des kératinocytes bulbaires en état de division permanent pendant les phases de croissances du cheveu. Le cheveu est constitué principalement de 85-90 % de protéines.

Avoir des cheveux sains et vigoureux tout au long de la vie est recherché par la plupart des femmes et des hommes.

A cet effet, il existe de nombreuses formulations de shampoings, après-shampoings, masques, huiles pour cheveux, etc., qui agissent directement sur les cheveux ou le cuir chevelu. Des compléments alimentaires ingérés par voie orale sont également utilisés pour le soin des cheveux, par exemple pour diminuer la chute des cheveux ou favoriser leur croissance.

Les compléments alimentaires disponibles sur le marché sont majoritairement à base de vitamine B et contiennent parfois de la vitamine A, de la vitamine E ou de la vitamine C. Outre ces vitamines, les compléments alimentaires contiennent généralement des acides aminés soufrés et/ou du zinc.

Les vitamines B5 et B8 sont par exemple utilisées pour prévenir la chute des cheveux, les vitamines B2 et B6 pour prévenir et traiter les pellicules et la vitamine B3 pour stimuler la microcirculation. Par ailleurs, les acides aminés soufrés, tels que la méthionine et la cystéine, sont utilisés pour favoriser la croissance des cheveux.

Enfin, le zinc est généralement utilisé pour favoriser la pousse des cheveux. Le zinc est en effet nécessaire à la synthèse de la kératine. Son utilité a également été démontrée dans la prévention de l'hyperséborrhée et de l'alopécie.

Pour autant les consommateurs sont toujours à la recherche d'actifs ou d'associations d'actifs efficaces pour améliorer la qualité de leur chevelure et cela concerne aussi bien les femmes que les hommes, de tout âge.

### Résumé de l'invention

La présente invention est définie par les revendications.

La présente demande repose sur la mise en évidence par les inventeurs que l'administration par voie orale de vitamine D et d'au moins un acide gras polyinsaturé provenant d'huile de poisson et d'huile de pépins de cassis, associés à d'autres actifs, permet d'améliorer la qualité de la chevelure.

Ainsi, de manière surprenante, les inventeurs ont mis en évidence que des compositions cosmétiques destinées à une administration par voie orale qui ne sont pas à base de vitamine B, mais de vitamine D, sont utiles pour améliorer la qualité de la chevelure.

Les inventeurs ont notamment mis en œuvre au moins une composition cosmétique pouvant se présenter sous forme de capsules molles qui comprend une association d'actifs comprenant des acides gras polyinsaturés présents sous forme d'huile de poisson et d'huile de pépins de cassis, de la vitamine D sous forme de vitamine D3 , de la vitamine E, de la vitamine C, un caroténoïde, ledit caroténoïde étant du lycopène, de préférence présent sous forme d'un extrait de tomates, et du gluconate de zinc.

L'administration de la composition ci-dessus, notamment à raison de deux capsules par jour, permet d'améliorer la qualité de la chevelure.

La demande décrit ainsi l'utilisation cosmétique, par voie orale, d'une association d'actifs comprenant un moins un acide gras polyinsaturé, tel que défini plus haut, et au moins de la vitamine D, pour améliorer la qualité de la chevelure, en particulier de l'homme et de la femme.

La demande décrit particulièrement l'utilisation cosmétique ci-dessus, dans laquelle l'association d'actifs est mise en œuvre au sein d'une composition cosmétique adaptée pour une administration par voie orale.

La demande décrit également une composition cosmétique destinée à une administration par voie orale comprenant :
(i) au moins un acide gras polyinsaturé mis en œuvre sous forme d'une huile de poisson et d'une huile de pépins de cassis, la teneur totale en acide gras polyinsaturé étant comprise entre 1% et 80 % en poids, par rapport au poids total de la composition cosmétique,
(ii) de la vitamine D en une teneur comprise entre 0,00001% et 0,15% en poids, par rapport au poids total de la composition,
(iii) du zinc, de préférence sous forme de gluconate de zinc, en une teneur comprise entre 0,001% et 30% en poids, par rapport au poids total de la composition,
(iv) de la vitamine E en une teneur comprise entre 0,001% et 10% en poids, par rapport au poids total de la composition,
(v) de la vitamine C en une teneur comprise entre 0,001% et 30% en poids, par rapport au poids total de la composition, et
(v) un caroténoïde, ledit caroténoïde étant du lycopène, la teneur totale en caroténoïde étant comprise entre 0,01% et 6% en poids, par rapport au poids total de la composition ; ladite composition ne comprenant pas de vitamine B.

La présente demande décrit également un procédé cosmétique pour améliorer la qualité de la chevelure, comprenant l'administration par voie orale à un individu d'une composition cosmétique destinée à une administration par voie orale telle que définie ci-dessus.

### Description détaillée

La demande décrit donc l'utilisation cosmétique par voie orale d'une association d'actifs telle que définie ci-dessus , pour améliorer la qualité de la chevelure, en particulier chez l'homme au-delà de 30 ans ou chez la femme.

De manière préférée tel que décrit dans la demande, l'utilisation cosmétique par voie orale d'une association d'actifs telle que définie ci-dessus est caractérisée en ce que l'amélioration de la qualité de la chevelure comprend l'amélioration de la brillance de la chevelure et/ou l'amélioration de la coiffabilité de la chevelure et/ou la diminution de la chute des cheveux et/ou l'amélioration de la croissance de la fibre capillaire, en particulier de fibres capillaires épaisses, et/ou l'amélioration du volume de la chevelure et/ou l'amélioration de la qualité de la fibre capillaire.

Par « améliorer la brillance de la chevelure », on entend désigner selon la présente invention le fait de favoriser une moindre usure et/ou d'améliorer la réparation des écailles de la cuticule, afin d'homogénéiser la surface du cheveu et favoriser la réflexion de la lumière De manière préférentielle et selon l'invention, les différents paramètres tels que définis ci-avant relatifs à l'amélioration de la brillance de la chevelure peuvent être évalués au moyen d'un questionnaire à choix multiples rempli par des hommes et/ou des femmes dans le cadre d'une étude observationnelle qui peut être réalisée chez des dermatologues. Un effet sera considéré comme significatif à partir du moment où au moins 50% des hommes et/ou des femmes auront perçu un effet positif. La brillance de la chevelure peut également être mesurée à l'aide d'un chromamètre et se définit par le paramètre L* dans le système CIE Lab. Le système CIE Lab (plus précisément L*a*b*) est un modèle de représentation des couleurs développé en 1976 par la Commission Internationale de l'Eclairage (CIE).

Par « améliorer la coiffabilité de la chevelure », on entend désigner selon la demande un cheveu plus facile à peigner et/ou à brosser, par exemple en renforçant, en resserrant ou en lissant les écailles des cheveux. En effet, des écailles disjointes ou abimées s'accrochent avec les écailles des cheveux voisins provoquant l'emmêlement de la chevelure qui favorise ensuite l'apparition de nœuds rendant le coiffage plus difficile. Par « améliorer la coiffabilité de la chevelure », on entend également une meilleure tenue de la coiffure après pose de bigoudis, brushing, coloration, décoloration, défrisage ou frisage.

Par « vieillissement du cheveu » on entend désigner selon la demande un changement d'aspect de la fibre, par exemple un cheveu fin, terne, sans tenue (c'est-à-dire mou), sans éclat et cassant facilement au peignage, des cheveux qui ont tendance à chuter, qui se renouvellent moins vite, dont le réseau de collagène qui les soutiennent est altéré par la libération importante de collagénase et la désorganisation de son réseau, dont les parties dermique et épithéliale se rigidifient par la présence de nombreux produits de glycation (Monnier VM, 1983), dont la synthèse de sébum se réduit entraînant une sécheresse du cuir chevelu et augmentant d'autant l'aspect terne et sans éclat du cheveu, dont l'épiderme et le derme du scalp sont aussi soumis à une désorganisation du réseau de collagène, une importante apparition de produits de glycation qui rigidifient le derme autour du follicule pileux entraînant des effets négatifs pour les follicules résidents, comme une miniaturisation progressive des bulbes, et un environnement défavorable à l'implantation de nouveaux cheveux.

On entend également par « vieillissement du cheveu » une diminution de la densité capillaire, ainsi que du diamètre des cheveux se traduisant par une diminution de la couverture du scalp.

La présente demande décrit également l'utilisation cosmétique par voie orale d'une association d'actifs telle que définie ci-dessus pour lutter contre le vieillissement du cheveu et du tissu périfolliculaire en contact, en particulier chez l'homme au-delà de 30 ans ou chez la femme.

Par « prévenir la chute » ou « diminuer la chute des cheveux », on entend selon la demande une diminution du pourcentage de cheveux en phase télogène et/ou une diminution du pourcentage de cheveux en phase de chute.

Une diminution du pourcentage de cheveux en phase télogène est mesurable avec le Trichoscan® en se basant sur le fait que les cheveux en phase télogène ne poussent plus, contrairement aux cheveux en phase anagène. Une diminution de 5% du nombre de cheveux en phase télogène peut être considérée comme significative.

Une diminution du pourcentage de cheveux en phase de chute est mesurable à l'aide du trichogramme ou du Trichoscan®, ou par recueil de cheveux au coiffage ou après la douche en se basant sur le fait que les cheveux en phase de chute sont le reflet visible des cheveux précédemment engagés en phase d'arrêt de la croissance, c'est-à-dire en phase télogène, et représentent la chute telle que réellement perçue par un individu affecté par une chute anormalement élevée de cheveux. Une diminution de 5% du nombre de cheveux en phase de chute peut être considérée comme significative.

Par « amélioration de la croissance des fibres capillaires, on entend désigner selon la demande, l'augmentation de la pousse de cheveux. De manière préférentielle selon la demande, l'amélioration de la croissance des fibres capillaires peut être évaluée au moyen d'un questionnaire à choix multiples rempli par des hommes et/ou des femmes, dans le cadre d'une étude observationnelle qui peut être réalisée chez des dermatologues. Un effet sera considéré comme significatif à partir du moment où au moins 50% des hommes et/ou des femmes auront perçu un effet positif. La croissance des fibres capillaires peut également être mesurée après coloration ou décoloration d'une mèche. La mesure de la distance entre la racine et la limite de la coloration ou décoloration permet d'évaluer la croissance des fibres capillaires.

Par « amélioration de la croissance de fibres capillaires épaisses », on entend désigner selon l'invention, l'augmentation de la pousse de cheveux avec un diamètre supérieur à 40 µm. Le diamètre des cheveux peut avantageusement être mesuré à l'aide du Trichoscan® équivalent automatisé du trichogramme où l'œil humain a été remplacé par un logiciel d'analyse d'image (Gasmueller, 2009). Une augmentation de 5% du nombre de cheveux avec un diamètre supérieur à 40 µm peut être considérée comme significative.

De manière encore préférée décrite ici, l'amélioration du volume de la chevelure comprend l'augmentation du diamètre de la fibre capillaire, et/ou l'augmentation de la densité, et/ou la limitation de l'affinement de la fibre capillaire.

Par « augmenter le diamètre de la fibre capillaire », on entend désigner selon la demande une augmentation du nombre de cheveux ayant un diamètre supérieur à 40 µm. Une augmentation de 5% du nombre de cheveux avec un diamètre supérieur à 40 µm peut être considéré comme significative.

Par « augmenter la densité », on entend selon la demande un plus grand nombre de cheveux par cm². La densité des cheveux est également mesurable avec le Trichoscan®. Avec cet appareil, une densité inférieure à 250 cheveux par cm² est considérée comme une densité faible. Une augmentation de 5% de la densité peut être considérée comme cliniquement significative et visible.

Par « affinement des cheveux », on entend désigner selon la demande une diminution du diamètre des cheveux en dessous de 40 µm. Le diamètre des cheveux peut être avantageusement mesuré à l'aide du Trichoscan® équivalent automatisé du trichogramme où l'œil humain a été remplacé par un logiciel d'analyse d'image (Gasmueller, 2009). Par ailleurs, en dessous de 40 µm les cheveux sont difficilement visibles à l'œil nu. L'affinement des cheveux peut donc être perçu. Une diminution de 5% du nombre de cheveux ayant un diamètre en dessous de 40 µm peut être considérée comme significative.

Par « améliorer le volume de la chevelure », on entend notamment désigner selon la demande une augmentation du diamètre des cheveux associée à une diminution de l'hétérogénéité du diamètre des cheveux, et à une augmentation de la densité.

L'hétérogénéité du diamètre des cheveux peut être mesurée au moyen d'une loupe binoculaire, par exemple en triant une mèche de 50 à 100 cheveux coupés (ou bien épilés), en les classant, par exemple en quatre catégories (très fins, fins, épais et très épais), et en calculant le pourcentage de cheveux dans chaque catégorie. L'hétérogénéité du diamètre des cheveux est alors évaluée en comparant l'évolution de ces pourcentages au cours du temps.

De manière également préférée décrite ici, l'amélioration de la qualité de la fibre capillaire comprend la résistance à la traction des cheveux, et/ou la prévention et/ou la lutte contre les cheveux mous et/ou cassants et/ou ternes et/ou fourchus et/ou fragilisés et/ou sensibilisés et/ou secs, et/ou l'amélioration de la douceur et de la vigueur des fibres capillaires.

Par « prévenir et/ou lutter contre les cheveux mous et/ou cassants et/ou ternes et/ou fourchus et/ou fragilisés et/ou sensibilisés et/ou secs », on entend désigner selon la demande une amélioration globale de la structure de la tige pilaire et notamment de la cuticule, couche la plus externe du cheveu. La qualité de la cuticule peut être évaluée, par exemple au moyen d'un microscope, en notant la présence d'écailles resserrées ou disjointes, d'écailles intactes on non et/ou d'écailles cassées, voire manquantes. Une diminution de 5% du nombre d'écailles disjointes, cassées ou manquantes peut être considérée comme significative.

Par « améliorer la douceur », on entend désigner selon l'invention une amélioration de l'état des écailles de la cuticule et plus particulièrement de leur cohésion. Des écailles disjointes, cassées ou manquantes rendent les cheveux rêches au toucher. L'amélioration de la douceur peut être évaluée, par exemple au moyen d'un microscope, en notant la présence d'écailles disjointes, cassées ou manquantes. Une diminution de 5% du nombre d'écailles disjointes, cassées ou manquantes peut être considérée comme significative.

Par « améliorer la résistance à la traction des cheveux » et « améliorer la vigueur des fibres capillaires », on entend designer selon l'invention la solidité des cheveux qui peut se mesurer par le test à la traction. Cette mesure de détermination des propriétés mécaniques en traction du cheveu peut être réalisée à l'aide d'un outil commercial, le MTT600 (mini Tensile Tester) de la société Dia Stron (http://www.diastron.com/). Une augmentation de 5% de la force nécessaire pour rompre le cheveu est considérée comme significative.

Les différents paramètres tels que définis ci-dessus peuvent être évalués au moyen d'un questionnaire à choix multiples rempli par des hommes et/ou des femmes dans le cadre d'une étude observationnelle qui peut être réalisée chez des dermatologues. Un effet sera considéré comme significatif à partir du moment où au moins 50% des hommes et/ou des femmes auront perçu un effet positif.

Sont particulièrement concernés par les utilisations selon l'invention les hommes de plus de 30 ans et les femmes.

Par « homme », on entend, au sens de la présente invention, la population masculine humaine.

### Acides gras polvinsaturés

On entend par "acide gras polyinsaturé" au sens de la présente invention, un acide gras comprenant au moins deux doubles liaisons. Il s'agit plus particulièrement d'acides gras à longues chaînes, c'est-à-dire possédant au moins 14 atomes de carbone.

Les acides gras polyinsaturés peuvent être sous forme acide, sous forme de triglycérides ou sous forme d'esters de méthyle ou d'éthyle.

Les acides gras polyinsaturés comprennent notamment les acides gras ω-3, les acides gras ω-6 et les acides gras ω-9, caractérisés par la position de l'insaturation la plus proche du groupe méthyle terminal, et leurs mélanges.

Par la suite, on utilise indifféremment les termes « ω » et « oméga ».

Conviennent tout particulièrement à la demande les acides gras polyinsaturés comportant de 18 à 22 atomes de carbone, plus préférentiellement les acides gras ω-3, ω-6 et ω-9.

Parmi les acides gras polyinsaturés de la série ω-6, on peut citer en particulier l'acide linoléique à 18 atomes de carbone et deux insaturations ou LA (18:2, ω-6), l'acide γ-linolénique à 18 atomes de carbone et trois insaturations ou GLA (18:3, ω-6), l'acide dihomogamalinolénique à 20 atomes de carbone et 3 insaturations (20:3, ω-6), l'acide arachidonique, l'acide 5,8,11,14 éicosatétraénoïque (20:4, ω-6) et l'acide docosatétraenoique (22:4, ω-6).

Les acides gras polyinsaturés de la série ω-3 peuvent notamment être choisis parmi l'acide α-linolénique ou ALA (18:3, ω-3), l'acide stéaridonique ou SDA (18:4, ω-3), l'acide 5,8,11,14,17-eicosapentaénoïque ou EPA (20:5, ω-3), l'acide 4,7,10,13,16,19-docosahéxaénoïque ou DHA (22:6, ω-3), l'acide docosapentanoique ou DPA (22,5, ω-3) et l'acide n-butyl-5,11,14-eicosatriènonique.

Les acides gras polyinsaturés de la série ω-9 peuvent notamment être choisis parmi l'acide oléique (18:1, ω-9) et l'acide érucique (22 :1, ω-9).

Conviennent tout particulièrement à la demande l'acide α-linolénique, l'acide linoléique, l'acide γ-linolénique, l'acide stéaridonique, l'acide éicosapentaénoïque, l'acide docosahexaénoïque, l'acide oléique, un extrait les comprenant ou leurs combinaisons.

Selon une variante décrite dans la demande, le ou les acide(s) gras polyinsaturé(s) considéré(s) est (sont) utilisé(s) sous une forme isolée, c'est-à-dire après extraction de sa (leur(s)) source(s) d'origine.

La teneur totale en acide(s) gras polyinsaturé(s) dans une composition décrite ici peut varier de 1% à 80 % en poids, de préférence de 5% à 70 % en poids, plus préférentiellement de 10% à 60 % en poids, plus préférentiellement encore de 20% à 50 % en poids par rapport au poids total de la composition.

Par exemple, une composition décrite ici peut comprendre une teneur totale en acide(s) gras polyinsaturé(s) comprise entre 35% et 45% en poids.

Une composition décrite ici, telle que développée ci-après, peut comprendre le(s) acide(s) gras polyinsaturé(s) en une concentration ajustée pour que le(s) acide(s) gras polyinsaturé(s) soit (soient) administré(s) à une teneur allant de 100 mg/jour à 1500 mg/jour, de préférence de 200 mg/jour à 700 mg/jour, plus préférentiellement de 350 mg/jour à 550 mg/jour.

Par exemple, une composition décrite ici peut comprendre le(s) acide(s) gras polyinsaturé(s) en une concentration ajustée pour que le(s) acide(s) gras polyinsaturé(s) soit (soient) administré(s) à une teneur allant de 400 mg/jour à 500 mg/jour.

La source d'acide gras polyinsaturé peut être choisie parmi les huiles végétales, comme par exemple l'huile d'onagre, l'huile de bourrache, l'huile de pépin de cassis, l'huile d'echium, l'huile de chanvre, un extrait de la microalgue *Schizochytrium sp* ou leurs combinaisons. La source végétale d'acide gras polyinsaturé est de préférence l'huile de pépins de cassis.

La source d'acide gras polyinsaturé peut également être choisie parmi les huiles de poisson.

Les huiles végétales de noix, noisettes, amandes (Juglans regia), de coriandre et de soja (Glycina max), de colza (Brassica naptus), de chia, de lin et les huiles de poisson, par exemple, sont riches en acides gras polyinsaturés de la série ω-3.

Les acides gras polyinsaturés ω-3 peuvent également se trouver dans le zooplancton, les crustacés/mollusques et les poissons. Les huiles de poissons constituent la principale source industrielle d'EPA et de DHA. Les biomasses de microalgues peuvent aussi constituer une matière première d'extraction des acides gras polyinsaturés ω-3.

Ainsi, un acide gras polyinsaturé peut être mis en œuvre sous forme d'au moins une huile choisie parmi l'huile d'onagre, l'huile de bourrache, l'huile de pépins de cassis, l'huile de noix, l'huile de soja, l'huile de poisson, l'huile de tournesol, l'huile de germes de blé, l'huile de chanvre, l'huile de fénugrec, l'huile d'échium, l'huile d'argan, l'huile de son de riz, l'huile de sésame, l'huile d'amande, l'huile de noisette, l'huile de chia, l'huile de lin, l'huile d'olive, l'huile d'avocat, l'huile de carthame, l'huile de cameline, l'huile d'Inca Inchi, l'huile de fruits de la passion, l'huile de pépins de framboises, l'huile de graines de canneberge, l'huile de pépins de myrtille, l'huile de graines de Rubus chamaemorus Linné, l'huile d'argousier, l'huile de cumin, l'huile de pépins de kiwi, l'huile de coriandre, l'huile d'extrait de microalgues, l'huile d'extrait de zooplancton, l'huile d'extrait de crustacés et/ou mollusques, ou leur combinaisons.

L'huile d'extrait de microalgues est par exemple une huile d'extrait de *Schizochytrium sp.*

Dans la présente demande, il s'agit d'un acide gras polyinsaturé mis en oeuvre sous forme d'une huile de pépins de cassis et d'une huile de poisson.

Une composition décrite ici, telle que développée ci-après, peut comprendre une huile et/ou un extrait et/ou une biomasse dans une teneur comprise entre 20% et 99% en poids, de préférence entre 30% et 95 % en poids, plus préférentiellement entre 50% et 90 %, plus préférentiellement encore entre 60% et 85% par rapport au poids total de la composition.

Par exemple, une composition décrite ici peut comprendre une huile et/ou un extrait et/ou une biomasse dans une teneur comprise entre 80% et 85 % en poids par rapport au poids total de la composition.

Une composition décrite ici, telle que développée ci-après, peut comprendre une huile et/ou un extrait et/ou une biomasse en une concentration ajustée pour que ladite huile et/ou extrait et/ou biomasse soit administrée à une teneur allant de 100 mg/jour à 5000 mg/jour, de préférence de 150 mg/jour à 3000 mg/jour, plus préférentiellement de 200 mg/jour à 1000 mg/jour.

Par exemple, une composition décrite ici peut comprendre une huile et/ou un extrait et/ou une biomasse en une concentration ajustée pour que ladite huile et/ou extrait et/ou biomasse soit administrée à une teneur allant de 200 mg/jour à 500 mg/jour.

Ainsi, selon un mode de réalisation préféré décrit ici, une association d'actifs décrite ici comprend au moins une huile choisie parmi une huile de pépins de cassis et une huile de poisson.

Selon un mode de réalisation davantage préféré décrit ici, une association d'actifs décrite ici comprend une huile de pépins de cassis et une huile de poisson, de préférence dans une teneur comprise entre 80% et 85 % en poids, par exemple pour une administration à une teneur allant de 200 mg/jour à 500 mg/jour.

### Vitamine D

La vitamine D est de préférence choisie parmi la vitamine D3 (appelée également cholécalciférol), la vitamine D2 (ergocalciférol) et des mélanges de celles-ci. La vitamine D utilisée dans l'association d'actifs selon l'invention est de préférence la vitamine D3.

Une composition décrite ici, telle que développée ci-après, peut comprendre de la vitamine D dans une teneur comprise entre 0,00001% et 0,15 % en poids, de préférence entre 0,00005 % et 0,10 % en poids, et plus préférentiellement entre 0,0001% et 0,10% en poids, plus préférentiellement encore entre 0,0001% et 0,05% en poids par rapport au poids total de la composition.

Par exemple, une composition conforme à l'invention peut comprendre de la vitamine D dans une teneur comprise entre 0,0004% et 0,0009% en poids par rapport au poids total de la composition.

Une composition conforme à l'invention, telle que développée ci-après, peut comprendre de la vitamine D en une concentration ajustée pour qu'elle soit administrée à une teneur allant de 0,0001 mg/jour à 0,05 mg/jour, de préférence de 0,0005 mg/jour à 0,01 mg/jour, plus préférentiellement de 0,001 mg/jour à 0,005 mg/jour

Par exemple, une composition conforme à l'invention peut comprendre de la vitamine D en une concentration ajustée pour qu'elle soit administrée à une teneur allant de 0,002 mg/jour à 0,006 mg/jour.

### Actifs additionnels

Une association d'actifs décrite ici comprend également un ou plusieurs autres actif(s) cosmétique(s) additionnel(s).

Avantageusement, un tel actif cosmétique additionnel peut être destiné à renforcer l'effet cosmétique souhaité tel que décrit précédemment.

Bien entendu, l'homme du métier veillera à choisir les actifs additionnels ainsi que leur quantité de telle manière que les propriétés avantageuses de la composition décrite ici ne soient pas, ou substantiellement pas, altérées par l'adjonction envisagée.

A titre d'actif additionnel utilisable, on peut citer :
- les vitamines autres que la vitamine D, tels que les vitamines C et E, et la vitamine A par exemple;
- les antioxydants, tels que les curcuminoïdes ; les caroténoïdes, notamment choisis parmi le β-carotène, l'astaxanthine, la zéaxanthine, la lutéine ou les composés en contenant comme les baies de goji ; des composés polyphénols, les flavonoïdes tels que les catéchines ; les proanthocyanidines, les anthocyanines, les OPC (oligomères procyanidoliques) ; les ubiquinones ; les extraits de café contenant des polyphénols et/ou des diterpènes ; les extraits de chicorés ; les extraits de ginkgo biloba ; les extraits de raisins riches en proanthocyanidines ; les extraits de piment ; les extraits de soja ; le cacao ; la grenade ; l'Emblica ; le CoenzymeQ10 ; le sélénium,
- les minéraux, tels que le zinc, le calcium, le magnésium, le cuivre, le fer, l'iode, le manganèse, le sélénium, le chrome (III),
- les sucres,
- les acides aminés, notamment les acides aminés soufrés, tels que des précurseurs de glutathion, les acides aminés du sélénium, la citrulline,
- les phytostérols,
- le resvératrol,
- l'hespéridine, la néohesperidine,
- l'acide orthosilicique, le monométhylsilanetriol, et
- leurs mélanges.

Dans la présente demande, l'association d'actifs comprend au moins un composé choisi parmi une vitamine autre que la vitamine D, ladite vitamine autre que la vitamine D étant la vitamine C ou la vitamine E, un caroténoïde, ledit caroténoïde étant du lycopène, et du zinc.

L'association d'actifs décrite ici ne comprend pas de vitamine B, et éventuellement ne comprend pas d'acide aminé soufré ou, plus généralement, ne comprend pas d'acides aminés.

Dans un mode de réalisation avantageux décrit ici, l'association d'actifs décrite ici ne comprend pas de minéraux autres que le zinc.

Dans un autre mode de réalisation avantageux décrit ici, l'association d'actifs décrite ici ne comprend pas d'antioxydants autres que des caroténoïdes et/ou des vitamines, ou encore ne comprend pas d'antioxydants autres que le lycopène, la vitamine C et la vitamine E.

L'expression « l'association d'actifs ne comprend pas l'élément X » signifie ici que ladite association ne comprend pas ledit élément X autre que celui éventuellement apporté par les composés comprenant les actifs de l'association, tels qu'une huile végétale, une huile de poisson et/ou un extrait de tomate.

### Vitamines C et E

La présente demande décrit une association d'actifs comprenant au moins un acide gras polyinsaturé tel que décrit ci-dessus, un caroténoïde, ledit caroténoïde étant du lycopène, du zinc, de la vitamine D et au moins une autre vitamine choisie parmi la vitamine C et la vitamine E.

La vitamine E est de préférence sous la forme d'acétate de tocophérol.

Une composition décrite ici, telle que développée ci-après, peut comprendre de la vitamine C dans une teneur comprise entre 0,001% et 30 % en poids, de préférence entre 0,01 % et 25 % en poids, et plus préférentiellement entre 0,1% et 20% en poids, plus préférentiellement encore entre 1% et 15% en poids par rapport au poids total de la composition.

Par exemple, une composition décrite ici peut comprendre de la vitamine C dans une teneur comprise entre 2% et 3% en poids par rapport au poids total de la composition.

Une composition décrite ici, telle que développée ci-après, peut comprendre de la vitamine C en une concentration ajustée pour qu'elle soit administrée à une teneur allant de 5 mg/jour à 100 mg/jour, de préférence de 10 mg/jour à 90 mg/jour, plus préférentiellement de 20 mg/jour à 80 mg/jour.

Par exemple, une composition décrite ici peut comprendre de la vitamine C en une concentration ajustée pour qu'elle soit administrée à une teneur allant de 25 mg/jour à 30 mg/jour.

Une composition décrite ici, telle que développée ci-après, peut comprendre de la vitamine E dans une teneur comprise entre 0,001% et 10% en poids, de préférence entre 0,01% et 10 % en poids, et plus préférentiellement entre 0,1% et 5% en poids, plus préférentiellement encore entre 0,2% et 2% en poids par rapport au poids total de la composition.

Par exemple, une composition conforme à l'invention peut comprendre de la vitamine E dans une teneur comprise entre 0,4% et 0,5% en poids par rapport au poids total de la composition.

Une composition conforme à l'invention, telle que développée ci-après, peut comprendre de la vitamine E en une concentration ajustée pour qu'elle soit administrée à une teneur allant de 0,1 mg/jour à 20 mg/jour, de préférence de 1 mg/jour à 15 mg/jour, plus préférentiellement de 2 mg/jour à 12 mg/jour.

Par exemple, une composition conforme à l'invention peut comprendre de la vitamine E en une concentration ajustée pour qu'elle soit administrée à une teneur allant de 4 mg/jour à 6 mg/jour.

### Caroténoïde

Une association d'actifs décrite ici comprend au moins un caroténoïde, ledit caroténoïde étant du lycopène.

Par « caroténoïde », on entend dans le cadre de la présente demande, aussi bien un caroténoïde à activité provitaminique A, qu'un caroténoïde sans activité provitaminique A.

Bien entendu selon la demande,
le caroténoïde présent dans l'association d'actifs décrite ici comprend, voire est constitué par, le lycopène.

Le caroténoïde utilisé selon l'invention peut être d'origine naturelle ou synthétique. Par origine naturelle, on entend le caroténoïde, à l'état pur ou en solution quelle qu'en soit sa concentration dans ladite solution, obtenu à partir d'un élément naturel tel qu'un extrait végétal. Ainsi, lorsque le caroténoïde est le lycopène, on peut utiliser plus particulièrement un extrait de tomate.

Ainsi, selon un mode de réalisation préféré décrit ici, le caroténoïde mis en œuvre dans une association décrite ici est le lycopène, de préférence dans un extrait de tomate, plus préférentiellement un extrait de tomate riche en lycopène.

Un extrait de tomate riche en lycopène comprend par exemple au moins 5% de lycopène, plus préférentiellement au moins 7% de lycopène, plus préférentiellement encore au moins 10% de lycopène en poids par rapport au poids total de l'extrait.

Un extrait de tomate riche en lycopène comprend avantageusement au moins 10% de lycopène, en poids par rapport au poids total de l'extrait.

Par « origine synthétique », on entend le lycopène, à l'état pur ou en solution quelle qu'en soit sa concentration dans ladite solution, obtenu par synthèse chimique. Le lycopène pouvant être utilisé dans le cadre de la présente invention peut être sous forme chimique cis ou trans.

Lorsque le caroténoïde est d'origine naturelle, il peut être obtenu à partir de matériel végétal issu de plante entière cultivée *in vivo* ou issu de culture *in vitro.*

Par « cultivée *in vivo* » on entend toute culture de type classique c'est-à-dire en sol à l'air libre ou en serre, ou encore hors sol.

Par « culture *in vitro* », on entend l'ensemble des techniques connues de l'homme du métier qui permet de manière artificielle l'obtention d'un végétal ou d'une partie d'un végétal. La pression de sélection imposée par les conditions physico-chimiques lors de la croissance des cellules végétales *in vitro* permet d'obtenir un matériel végétal standardisé et disponible tout au long de l'année contrairement aux plantes cultivées *in vivo.*

Préférentiellement selon l'invention, on utilise un végétal issu de culture *in vivo.* Toute méthode d'extraction connue de l'homme du métier peut être utilisée pour préparer le caroténoïde utilisé selon l'invention.

Très préférentiellement, dans le cas du lycopène, on utilise un extrait de tomate riche en lycopène.

Le lycopène est également présent dans le melon, la goyave et le pamplemousse.

Le lycopène peut être en solution alcoolique, notamment éthanolique. Le caroténoïde peut également être en solution lipidique ou lipoalcoolique.

Le lycopène peut être en suspension aqueuse. Pour cela, on peut utiliser des formes hydrodispersibles, à froid ou à chaud.

Tout autre ingrédient plus complexe à base de lycopène peut également être utilisé pour la mise en oeuvre de l'invention.

Ainsi, on entend au titre d'ingrédient plus complexe par exemple une composition primaire comprenant le lycopène et une protéine de petit lait. Cette composition primaire est notamment décrite dans le document WO 01/91588. Cette composition primaire porte également le nom de lactolycopène. Il présente l'intérêt d'augmenter la biodisponibilité du lycopène et/ou d'être aisément formulable dans les compléments alimentaires (formes sachet, gélule, comprimé, dragée, capsule molle, ...).

Le lactolycopène peut notamment être vendu par la société Indena.

La quantité d'extrait utilisable selon la demande est bien entendu fonction de l'effet recherché et peut donc varier dans une large mesure.

La teneur totale en caroténoïde(s), de préférence en lycopène, dans une composition décrite ici peut varier de 0,01% à 6 % en poids, de préférence de 0,02% à 4% en poids, plus préférentiellement de 0,05% à 3% en poids, plus préférentiellement encore de 0,07% à 2% en poids, par rapport au poids total de la composition.

Par exemple, une composition décrite ici peut comprendre une teneur totale en caroténoïde(s), de préférence en lycopène, comprise entre 0,09% et 0,1% en poids par rapport au poids total de la composition.

Une composition décrite ici, telle que développée ci-après, peut comprendre au moins un caroténoïde, de préférence du lycopène, en une concentration totale en caroténoïde(s) ajustée pour une administration à une teneur allant de 0,1 mg/jour à 10 mg/jour, de préférence de 0,5 mg/jour à 8 mg/jour, plus préférentiellement de 0,8 mg/jour à 6 mg/jour.

Par exemple, une composition décrite ici peut comprendre au moins un caroténoïde, de préférence du lycopène, en une concentration ajustée pour qu'elle soit administrée à une teneur allant de 1 mg/jour à 1,5 mg/jour.

Ainsi, une association d'actifs décrite ici comprend avantageusement au moins une huile comprenant au moins un acide gras polyinsaturé, de la vitamine D et du lycopène isolé et/ou un extrait riche en lycopène.

Selon un mode de réalisation préféré décrit ici, une association d'actifs décrite ici comprend au moins un acide gras polyinsaturé mis en oeuvre sous forme de l'huile de poisson et de l'huile de pépins de cassis, de la vitamine D, du zinc, de la vitamine C, de la vitamine E et un caroténoïde étant du lycopène mis en oeuvre sous la forme d' un extrait de tomate.

### Zinc

L'association d'actifs décrite ici comprend également du zinc.

Par « zinc », on entend le zinc ou l'un de ses sels (acétate, chlorure, citrate, lactate, gluconate, lactate, oxyde, carbonate ou sulfate de zinc), en particulier les sels de Zn (II) et de préférence complexés par un ou plusieurs (poly)hydroxyacides, tel que le gluconate.

Selon un mode de réalisation particulier de l'invention, le zinc n'est pas un oxyde de zinc, mais un sel de zinc.

Dans la mesure où le produit selon l'invention est destiné à une mise en œuvre par voie orale chez un individu, les sels pouvant être mis en œuvre sont bien évidemment choisis pour leur totale innocuité.

Par Zn(II), on entend un atome de zinc de degré d'oxydation Zn2+.

Par (poly)hydroxyacide, on entend tout acide carboxylique qui comprend une chaine hydrocarbonée, linéaire ou ramifiée, saturée ou insaturée, de préférence saturée et/ou linéaire, comprenant de 1 à 10 atomes de carbone et de 1 à 9 groupes hydroxy, et comprenant de 1 à 4 groupes carboxyliques -C(O)-OH, dont au moins une desdites fonctions -C(O)-OH est sous la forme carboxylate -C(O)-O- complexée avec l'atome de Zn, de préférence Zn(II).

Plus particulièrement, le sel de zinc est complexé par deux groupes carboxylates tels que celui de formule (I) R-C(O)-O-Zn-O-C(O)-R', ainsi que ses solvates tels que les hydrates et leurs énantiomères, R et R' étant identiques ou différents et représentant un groupe (C1-C6)(poly)hydroxyalkyle.

De préférence, le composé de formule (I) est du gluconate de zinc.

La teneur en zinc dans une composition conforme à l'invention peut varier par exemple de 0,001% à 30% en poids, de préférence de 0,05% à 20% en poids, et plus préférentiellement de 0,1 % à 10% en poids par rapport au poids total de la composition.

Par exemple, une composition conforme à l'invention peut comprendre une teneur en zinc, comprise entre 0,5% et 0,7% en poids par rapport au poids total de la composition.

Une composition conforme à l'invention, telle que développée ci-après, peut comprendre du zinc, en une concentration ajustée pour qu'elle soit administrée à une teneur allant de 0,01 mg/jour à 300 mg/jour, de préférence de 0,1 mg/jour à 200 mg/jour, plus préférentiellement de 1 mg/jour à 100 mg/jour, plus préférentiellement encore de 3 mg/jour à 10 mg/jour.

Par exemple, une composition conforme à l'invention peut comprendre du zinc en une concentration ajustée pour qu'elle soit administrée à une teneur allant de 6 mg/jour à 8 mg/jour.

### Agent antioxydants

Une association d'actifs décrite ici peut comprendre, outre les actifs cités ci-dessus, un agent antioxydant autre que les caroténoïdes.

Les agents antioxydants sont connus de l'homme de l'art.

Les agents antioxydants peuvent par exemple être choisis parmi les curcuminoïdes ; des composés polyphénols, les flavonoïdes tels que les catéchines ; les proanthocyanidines, les anthocyanines, les OPC (oligomères procyanidoliques) ; les ubiquinones ; les extraits de café contenant des polyphénols et/ou des diterpènes ; les extraits de chicorés ; les extraits de ginkgo biloba ; les extraits de raisins riches en proanthocyanidines ; les extraits de piment ; les extraits de soja ; le cacao ; la grenade ; l'Emblica ; le CoenzymeQ10 ; le sélénium.

### Agent anti-inflammatoire

Une association d'actifs décrite ici peut aussi comprendre un agent anti-inflammatoire.

Les agents anti-inflammatoires sont connus de l'homme de l'art.

Un agent anti-inflammatoire décrit ici peut être choisi parmi les flavonoïdes tels que les catéchines, les proanthocyanidines, les anthocyanines, les oligomères procyanidoliques (OPC), les flavanones, par exemple l'hespéridine.

Dans un mode de réalisation particulier décrit ici, l'association d'actifs ne comprend pas d'agent anti-inflammatoire.

### Microorganisme probiotique et prébiotique

Selon un mode de réalisation particulier décrit ici, une composition décrite ici peut, en outre, comprendre au moins un microorganisme probiotique, au moins un prébiotique ou leurs combinaisons.

Des exemples spécifiques de microorganismes probiotiques convenant à la présente demande sont des microorganismes du genre *Bifidobacterium,* tels que *Bifidobacterium adolescentis, Bifidobacterium animalis, Bifidobacterium bifidum, Bifidobacterium breve, Bifidobacterium lactis, Bifidobacterium* /*ongum, Bifidobacterium infantis, Bifidobacterium pseudocatenulatum, Lactobacillus acidophilus (LC1, NCFB 1748)* ; des microorganismes du genre *Lactobacillus,* tels que *Lactobacillus amylovorus, Lactobacillus casei (Shirota), Lactobacillus rhamnosus (souche GG), Lactobacillus brevis, Lactobacillus crispatus, Lactobacillus delbrueckii (subsp. bulgaricus, lactis), Lactobacillus fermentum, Lactobacillus helveticus, Lactobacillus gallinarum, Lactobacillus gasseri, Lactobacillus johnsonii, Lactobacillus paracasei, Lactobacillus plantarum, Lactobacillus rhamnosus, Lactobacillus salivarius, Lactobacillus alimentarius, Lactobacillus curvatus, Lactobacillus casei subsp. casei, Lactobacillus sake,* des microorganismes du genre *Lactococcus,* tels que *Lactococcus lactis, Lactococcus lactis subspp. lactis ou cremoris,* des microorganismes du genre *Enterococcus,* tels que *Enterococcus faecalis ou Enterococcus faecium,* des microorganismes du genre *Leuconostoc,* tels que *Leuconostoc mesenteroides subsp. dextranicum,* des microorganismes du genre *Pediococcus,* tels que *Pediococcus acidilactici,* des microorganismes du genre *Sporolactobacillus,* tels que *Sporolactobacillus inulinus,* des microorganismes du genre *Streptococcus,* tels que *Streptococcus salvarius subsp. thermophilus, Streptococcus thermophilus,* des microorganismes du genre *Staphylococccus,* tels que *Staphylococccus carnosus, Staphylococcus xylosus,* des microorganismes du genre *Saccharomyces,* tels que *Saccharomyces cerevisiae ou* encore *Saccharomyces boulardii,* des microorganismes du genre *Bacillus,* tels que *Bacillus cereus var.* toyo, *Bacillus subtilis, Bacillus coagulans, Bacillus licheniformis,* des microorganismes du genre *Escherichia,* tels que *Escherichia coli strain nissle,* des microorganismes du genre *Propionibacterium,* tels que *Propionibacterium freudenreichii,* ou leurs combinaisons.

Les microorganismes peuvent être formulés à l'état de poudres, c'est-à-dire sous une forme sèche, ou sous forme de suspensions ou de solutions.

Plus particulièrement, il peut s'agir d'un microorganisme probiotique choisi parmi des microorganismes du genre *Lactobacillus sp.* et/ou *Bifidobacterium sp.,* l'une de leurs fractions et/ou l'un de leurs métabolites. A titre illustratif de ces microorganismes, on peut plus particulièrement citer les *Lactobacillus johnsonii, Lactobacillus rhamnosus, Lactobacillus paracasei, Lactobacillus casei, Bifidobacterium bifidum, Bifidobacterium breve, Bifidobacterium longum, Bifidobacterium animalis, Bifidobacterium lactis, Bifidobacterium infantis, Bifidobacterium adolescentis, Bifidobacterium pseudocatenulatum,* ou leurs combinaisons.

Les espèces convenant tout particulièrement sont *Lactobacillus johnsonii, Lactobacillus paracasei, Bifidobacterium adolescentis, Bifidobacterium longum* ou *Bifidobacterum Lactis* NCC 2818 (encore désignée Bb12 ATCC 27536) respectivement déposées suivant le traité de Budapest avec l'Institut Pasteur (28 rue du Docteur Roux, F-75024 Paris cedex 15) les 30/06/92, 12/01/99, 15/04/99, 15/04/99, 07/06/05 sous les désignations suivantes CNCM I-1225, CNCM I-2116, CNCM I-2168, CNCM I-2170 et CNCM I-3446, et l'espèce *Bifidobacterium longum* (BB536). La souche de *Bifidobacterium lactis* CNCM I-3446 peut être obtenue chez Hansen (Chr. Hansen A/S, 10-12 Boege Alle, P.O. Box 407, DK-2970 Hoersholm, Danemark).

Selon un mode de réalisation particulier décrit ici, la composition comprend au moins deux microorganismes différents, notamment probiotiques, et/ou des métabolites et/ou des fractions de ceux-ci. Ces microorganismes peuvent différer par leur nature par exemple bactérie et champignon, ou bien encore par leur famille, leur genre, leur espèce, ou seulement par leur souche.

Les prébiotiques convenant à la demande peuvent être choisis parmi les oligosaccharides produits à partir du glucose, galactose, xylose, maltose, sucrose, lactose, amidon, xylane, l'hémicellulose, l'inuline, des gommes, par exemple de type acacia, ou un de leurs mélanges. Plus particulièrement, l'oligosaccharide comprend au moins un fructo-oligosaccharide. Plus particulièrement, ce prébiotique peut comprendre un mélange de fructo-oligosaccharide et d'inuline.

Dans un mode de réalisation particulier décrit ici, l'association d'actifs ne comprend pas de microorganisme probiotique et/ou de prébiotique.

### Composition cosmétique destinée à une administration par voie orale et kits

Selon un aspect décrit ici, une association d'actifs décrite ici peut être mise en œuvre au sein d'une composition cosmétique adaptée pour une administration par voie orale.

La présente demande décrit particulièrement une composition cosmétique adaptée pour une administration par voie orale comprenant une association d'actifs telle que définie ci-dessus.

Par « composition cosmétique», on entend, par exemple, une composition nutritionnelle, par exemple un complément alimentaire, comprenant au moins une association d'actifs conforme à l'invention.

Les expressions «composition nutritionnelle », «composition nutraceutique » ou « composition cosméceutique » sont ici synonymes.

Par « complément alimentaire », on entend désigner ici une denrée alimentaire dont le but est de compléter le régime alimentaire normal et qui constitue une source concentrée de nutriments, c'est-à-dire vitamines et/ou minéraux, et/ou d'autres substances ayant un effet nutritionnel ou physiologique, seuls ou combinés, commercialisée sous forme de doses, à savoir les formes de présentation telles que les gélules, les pastilles, les comprimés, les capsules molles, les pilules et autres formes similaires, ainsi que les sachets de poudre à diluer, les ampoules de liquide, les flacons munis d'un compte-gouttes et les autres formes analogues de préparations liquides ou sous forme de boissons.

Une composition cosmétique préférée selon l'invention est un complément alimentaire.

Une composition cosmétique préférée décrite ici destinée à une administration par voie orale comprend :
- au moins un acide gras polyinsaturé, tel que défini plus haut, a teneur totale en acide gras polyinsaturé étant comprise entre 1% et 80 % en poids, de préférence entre 5% et 70% en poids, plus préférentiellement entre 10% et 60% en poids, plus préférentiellement encore entre 20% et 50% en poids,
- de la vitamine D en une teneur comprise entre 0,00001% et 0,15% en poids, de préférence entre 0,00005% et 0,1% en poids, plus préférentiellement entre 0,0001% et 0,1% en poids, plus préférentiellement encore entre 0,0001% et 0,05% en poids,
- du zinc en une teneur comprise entre 0,001% et 30% en poids, de préférence entre 0,05% et 20% en poids, plus préférentiellement entre 0,1% et 10% en poids, plus préférentiellement encore entre 0,2% et 5% en poids, le zinc étant de préférence sous la forme de gluconate de zinc,
- de la vitamine E en une teneur comprise entre 0,001% et 10% en poids, de préférence entre 0,01% et 10% en poids, plus préférentiellement entre 0,1% et 5% en poids, plus préférentiellement encore entre 0,2% et 2% en poids,
- de la vitamine C en une teneur comprise entre 0,001% et 30% en poids, de préférence entre 0,01% et 25% en poids, plus préférentiellement entre 0,1% et 20% en poids, plus préférentiellement encore entre 1% et 15% en poids, et
- au moins un caroténoïde, tel que défini plus haut, a teneur totale en caroténoïde étant comprise entre 0,01% et 6% en poids, de préférence entre 0,02% et 4% en poids, plus préférentiellement entre 0,05% et 3% en poids, plus préférentiellement encore entre 0,07% et 2% en poids,
les pourcentages étant donnés en poids par rapport au poids total de la composition cosmétique.

Un autre exemple de composition cosmétique préférée décrite ici destinée à une administration par voie orale consiste en :
- au moins un acide gras polyinsaturé tel que défini plus haut, la teneur totale en acide gras polyinsaturé étant comprise entre 1% et 80 % en poids, de préférence entre 5% et 70% en poids, plus préférentiellement entre 10% et 60% en poids, plus préférentiellement encore entre 20% et 50% en poids,
- de la vitamine D en une teneur comprise entre 0,00001% et 0,15% en poids, de préférence entre 0,00005% et 0,1% en poids, plus préférentiellement entre 0,0001% et 0,1% en poids, plus préférentiellement encore entre 0,0001% et 0,05% en poids,
- du zinc en une teneur comprise entre 0,001% et 30% en poids, de préférence entre 0,05% et 20% en poids, plus préférentiellement entre 0,1% et 10% en poids, plus préférentiellement encore entre 0,2% et 5% en poids, le zinc étant de préférence sous la forme de gluconate de zinc,
- de la vitamine E en une teneur comprise entre 0,001% et 10% en poids, de préférence entre 0,01% et 10% en poids, plus préférentiellement entre 0,1% et 5% en poids, plus préférentiellement encore entre 0,2% et 2% en poids,
- de la vitamine C en une teneur comprise entre 0,001% et 30% en poids, de préférence entre 0,01% et 25% en poids, plus préférentiellement entre 0,1% et 20% en poids, plus préférentiellement encore entre 1% et 15% en poids, et
- au moins un caroténoïde, tel que défini plus haut, la teneur totale en caroténoïde étant comprise entre 0,01% et 6% en poids, de préférence entre 0,02% et 4% en poids, plus préférentiellement entre 0,05% et 3% en poids, plus préférentiellement encore entre 0,07% et 2% en poids,
- optionnellement au moins un excipient, de préférence choisi parmi le monostéarate de glycérol, la cire d'abeille, la lécithine de soja ou leurs combinaisons,
les pourcentages étant donnés en poids par rapport au poids total de la composition cosmétique.

Une telle composition cosmétique adaptée pour une administration par voie orale, de préférence un complément alimentaire, peut présenter les teneurs suivantes :

| **Actif** | **% en poids par rapport au poids total de la composition** |
|---|---|
| Acide(s) gras polyinsaturé(s) (sous la forme d'huile de poisson et huile de pépins de cassis) | 35 à 37 (dont oméga 3 et oméga 6) |
| Vitamine D3 | 0,0004 à 0,0006 |
| Vitamine C | 2,4 à 2,5 |
| Vitamine E | 0,4 à 0,5 |
| Caroténoïde(s) (lycopène) | 0,09 à 0,10 |
| Zinc (de préférence sous forme de gluconate de zinc) | 0,6 à 0,7 |

Une composition conforme à l'invention comprend un véhicule physiologiquement ou cosmétiquement acceptable.

Une association d'actifs et une composition décrites ici permettent, de par leur administration par voie orale, d'améliorer la qualité de la chevelure.

L'invention ne se rapporte pas au domaine thérapeutique.

L'association d'actifs décrite ici ou la composition décrite ici est administrée par voie orale.

Les associations d'actifs et compositions décrites ici, destinées à l'administration par voie orale, peuvent notamment comprendre l'intégralité ou une partie seulement de la dose journalière.

Dans le cas de compositions convenant à une administration par voie orale, on privilégie l'utilisation d'un support ingérable. Le support ingérable peut être de nature diverse selon le type de composition considérée.

Pour l'ingestion, de nombreuses formes de réalisation de compositions orales et notamment de compléments alimentaires sont possibles.

La formulation de telles compositions peut être réalisée par tout procédé usuel connu de l'homme de l'art.

Ainsi, une composition conforme à l'invention peut de préférence prendre la forme d'une dragée, d'une gélule, d'une suspension, d'un gel, d'une émulsion, d'une solution buvable, d'un comprimé à avaler ou à croquer, d'une capsule, notamment d'une capsule molle ou dure, d'un granulé à dissoudre, d'un sirop, d'une tablette ou d'une ampoule buvable.

Elle peut de préférence se présenter sous la forme d'une capsule, molle ou dure, de préférence sous forme d'une capsule molle.

En particulier, une association d'actifs conforme à l'invention peut être mise en œuvre dans toutes formes de compléments alimentaires ou des poudres compactées ou non. Les poudres peuvent être diluées à l'eau ou dans du soda. Selon un mode préféré de réalisation, une composition conforme à l'invention administrée par voie orale peut être formulée sous forme de dragée, de gélule, de gel, d'émulsion, de comprimé, de capsule, d'hydrogel, de poudre compactée ou non, de suspension ou solution liquide.

Les compositions par voie orale peuvent se présenter soit sous forme anhydre, soit sous forme aqueuse.

Une association d'actifs conforme à l'invention peut être formulée avec les excipients usuels pour de telles compositions orales, tels que des compléments alimentaires, à savoir notamment des composants gras et/ou aqueux, des agents humectants, épaississants, conservateurs, des agents de texture, de saveur et/ou d'enrobage, des antioxydants, des conservateurs et des colorants usuels dans le domaine de l'alimentaire.

Bien entendu l'homme du métier veillera à choisir les éventuels excipients et agents de formulation et/ou leur quantité de telle manière que les propriétés avantageuses de l'association décrite ici ou de la composition comprenant l'association décrite ici ne soient pas ou substantiellement pas altérées par l'adjonction envisagée.

Les agents de formulation et excipients pour composition orale, et notamment pour compléments alimentaires sont connus dans ce domaine et ne font pas ici l'objet d'une description détaillée.

La demande décrit également un kit ou un produit de combinaison cosmétique pour une utilisation simultanée, séparée ou étalée dans le temps, de préférence un complément alimentaire, comprenant une première composition comprenant une partie des composés formant l'association d'actifs décrite ici et une deuxième composition comprenant au moins l'autre partie des composés formant ladite association d'actifs.

Ce complément peut être formulé de telle façon que les deux compositions sont sous les mêmes formes ou sous des formes différentes, par exemple choisies parmi celles citées plus haut. Un tel kit peut notamment être présenté dans un seul et même emballage.

### Utilisation cosmétique de l'association de principes actifs ou d'une composition les comprenant

La présente demande décrit particulièrement l'utilisation cosmétique, par voie orale, d'une association d'actifs telle que définie ci-dessus comprenant au moins un acide gras polyinsaturé et de la vitamine D, pour améliorer la qualité de la chevelure.

La présente demande décrit l'utilisation telle que définie ci-dessus, dans laquelle ledit au moins un acide gras polyinsaturé est mis en œuvre sous forme d'une huile de pépins de cassis et d'une huile de poisson et / ou ladite vitamine D est choisie parmi la vitamine D3 et la vitamine D2, de préférence la vitamine D3.

Dans un mode de réalisation particulièrement avantageux décrit ici, la présente demande décrit l'utilisation telle que définie ci-dessus, caractérisée en ce que l'association d'actifs comprend, à titre d'actif additionnel, au moins un composé choisi parmi une vitamine autre que la vitamine D, un caroténoïde, du zinc ou leurs combinaisons.

La présente demande décrit notamment l'utilisation telle que définie ci-dessus, dans laquelle l'association d'actifs est mise en œuvre au sein d'une composition cosmétique adaptée pour une administration par voie orale.

Les teneurs sont variables selon la forme de la composition cosmétique dans laquelle est mise en œuvre l'association d'actifs décrits ici.

Les teneurs dans la composition cosmétique, la forme de la composition cosmétique et les doses journalières administrées sont notamment telles que définies ci-dessus.

Il est entendu dans le cadre de la présente invention que « l'utilisation cosmétique par voie orale » recouvre l'utilisation de compositions administrées par voie orale, ces compositions étant par exemple sous forme de complément alimentaire. Ces compositions produisent un effet, au niveau de la chevelure, sur le plan esthétique et du confort, ou encore à visée beauté, par exemple en vue de la protéger, de la maintenir en bon état, et notamment de l'embellir, en améliorant la qualité de la chevelure.

### Procédé

La demande décrit également un procédé cosmétique pour améliorer la qualité de la chevelure, comprenant l'administration par voie orale à un individu d'une association d'actifs telle que définie ci-dessus, d'une composition cosmétique destinée à une administration par voie orale telle que définie ci-dessus.

Les procédés selon l'invention présentent les caractéristiques de procédés cosmétiques dans la mesure notamment où ils permettent d'améliorer l'esthétique de la chevelure. En outre, une association d'actifs ou une composition telle que par exemple un complément alimentaire selon l'invention peut être utilisée quotidiennement pendant plusieurs mois, sans prescription médicale. La présente invention se situe donc clairement en dehors du champ thérapeutique.

Avantageusement, l'application d'un procédé décrit ici confère les avantages indiqués précédemment comme étant associés à la mise en œuvre d'une association d'actifs ou d'une composition décrite ici, et peut notamment améliorer la brillance de la chevelure et/ou l'améliorer la coiffabilité de la chevelure et/ou diminuer la chute des cheveux et/ou améliorer la croissance de la fibre capillaire et/ou améliorer le volume de la chevelure et/ou améliorer la qualité de la fibre capillaire.

Un procédé cosmétique décrit ici peut être mis en œuvre, notamment par administration d'une composition cosmétique telle que définie ci-dessus.

Un procédé de l'invention peut être mis en œuvre de façon journalière par exemple, à raison par exemple d'une administration unique par jour ou d'une administration deux fois par jour, par exemple une fois le matin et une fois le soir, ou trois fois par jours, notamment à chaque repas.

Un procédé cosmétique selon l'invention peut être mis en œuvre, par exemple par administration journalière d'une composition formulée par exemple sous forme de dragée, gélule, suspension, gel, émulsion, solution buvable, comprimé à avaler ou à croquer, capsule, notamment capsule molle ou dure, granulé à dissoudre, sirop, tablette ou ampoule buvable, en quantité et nombre adéquats, selon leur forme.

Une quantité efficace d'une association d'actifs décrite ici peut être administrée en une dose unique par jour ou en doses fractionnées sur la journée, par exemple deux à trois fois par jour.

Un procédé selon l'invention peut avantageusement comprendre une administration unique. Un procédé cosmétique peut être mis en œuvre sur une période de temps variant d'une semaine à plusieurs semaines, voire plusieurs mois, cette période pouvant par ailleurs être répétée après des périodes de non traitement, pendant plusieurs mois voire plusieurs années.

A titre d'exemple, l'administration d'une association d'actifs décrite ici peut être effectuée à raison de deux fois par jour, généralement sur une durée prolongée d'au moins quatre semaines, voire quatre à quinze semaines, comprenant ou non une ou plusieurs périodes d'interruption ou étant répétée après une période d'interruption.

Les ingrédients sont mélangés, avant leur mise en forme, dans l'ordre et dans des conditions facilement déterminées par l'homme de l'art.

Ainsi, les utilisations et/ou procédés cosmétiques décrits ici permettent d'améliorer la qualité de la chevelure, en particulier chez l'homme ou la femme. Les utilisations et/ou procédés cosmétiques décrits ici permettent particulièrement de protéger les cheveux vieillis, notamment chez l'homme de plus de 30 ans ou chez la femme.

Dans la description et les exemples qui suivent, les plages de valeurs libellées sous la forme « entre ... et ... » incluent les bornes inférieure et supérieure précisées.

D'autres caractéristiques et avantages de l'invention ressortiront mieux des exemples qui suivent, donnés à titre illustratif et non limitatif.

### Exemples : Composition pour voie orale sous forme de capsule molle

La composition pour voie orale a la formule donnée dans le tableau suivant :

| **Ingrédients** | **Quantité ingrédient** (en mg/ capsule) | **Pourcentage de l'actif** (en poids par rapport au poids total de la composition hors capsule) |
|---|---|---|
| Huile de poisson | 230,00 | 12,62 (acides gras polyinsaturés) |
| Huile de pépins de cassis | 230,00 | 23,98 (acides gras polyinsaturés) |
| Vitamine D3 | 0,12 | 0,00053 |
| Vitamine E | 4,10 | 0,43 |
| Vitamine C | 19,50 | 2,46 |
| Extrait de tomate | 5,50 | 0,097 (lycopène) |
| D-Gluconate de zinc | 25,75 | 0,62 (zinc) |

| **Excipients :** | | |
|---|---|---|
| Mono-stéarate de glycérol | 30,03 | |
| Cire d'abeille | 10,00 | |
| Lécithine de soja | 10,00 | |

Calcul de la répartition en acides gras des huiles utilisées :

### (1) Huile de poisson :

Une capsule molle apporte 71,3 mg d'oméga 3 dont :
- 34,5 mg d'EPA (acide 5,8,11,14,17-eicosapentaénoïque) (oméga 3),
- 20,7 mg de DHA (acide 4,7,10,13,16,19-docosahéxaénoïque) (oméga 3).

### (2) Huile de pépins de cassis :

Une capsule molle apporte 135,47 mg d'acides gras polyinsaturés dont :
- 80,73 mg de LA (acide linolénique) (oméga 6),
- 26,91 mg de GLA (acide **γ**-linolénique) (oméga 6),
- 22,77 mg d'ALA (acide **α**-linolénique) (oméga 3),
- 5,06 mg de SDA (acide stéaridonique) (oméga 3).

La capsule molle a par exemple la formule suivante :

| **Capsule** | |
|---|---|
| Gélatine de poisson | 127,776 |
| Glycérol | 56,893 |
| Oxyde de fer | 2,051 |
| Dioxyde de titanium | 2,921 |
| Eau purifiée | 21,082 |

Une capsule molle selon l'invention peut être préparée de la façon suivante.

L'huile de poisson, l'huile de pépins de cassis, la vitamine E, la vitamine C, l'extrait de tomate, la vitamine D3, le gluconate de zinc et les excipients sont mélangés en présence d'azote. Le mélange est ensuite homogénéisé, puis encapsulé dans la capsule molle constituée de gélatine de poisson, de glycérol, d'oxyde de fer, de dioxyde de titanium et d'eau purifiée.

La posologie est par exemple de 1 à 4 capsules molles par jour, de préférence 2 capsules molles /jour.

Cette composition permet notamment :
- l'amélioration de la brillance de la chevelure, en particulier en favorisant la pousse de cheveux brillants,
- l'amélioration de la coiffabilité de la chevelure,
- la limitation de la chute des cheveux, en particulier en favorisant l'ancrage du cheveu,
- l'amélioration de la croissance de la fibre capillaire, en particulier en participant à la croissance normale du cheveu et en favorisant l'épaisseur de la chevelure, notamment par la pousse de cheveux épais,
- l'amélioration du volume de la chevelure, en particulier en favorisant l'abondance capillaire, en augmentant la masse capillaire et la densité des cheveux, en augmentant le diamètre des cheveux, en prévenant et/ou limitant la formation de cheveux fins,
- l'amélioration de la croissance et de la synthèse d'un cheveu de qualité, en particulier en prévenant et/ou limitant la formation de cheveux mous et/ou de cheveux ternes, en améliorant la souplesse des cheveux, en améliorant la force des cheveux et en favorisant la qualité du scalp (via la mesure de la microcirculation).

## Revendications

1. Utilisation cosmétique, par voie orale, d'une composition cosmétique consistant en au moins un acide gras polyinsaturé mis en œuvre sous forme d'une huile de poisson et d'une huile de pépins de cassis, de la vitamine D, un caroténoïde, ledit caroténoïde étant du lycopène, du zinc, de la vitamine E, de la vitamine C et, optionnellement, au moins un excipient, pour améliorer la brillance de la chevelure et/ou améliorer la croissance de fibres capillaires épaisses et/ou améliorer la douceur et la vigueur des fibres capillaires, ladite composition cosmétique ne comprenant pas de vitamine B.

2. Utilisation selon la revendication 1, **caractérisée en ce que** la vitamine D est choisie parmi la vitamine D3 et la vitamine D2, de préférence la vitamine D3.

3. Utilisation selon la revendication 1 ou 2, **caractérisée en ce que** le lycopène est mis en œuvre sous la forme d'un extrait de tomate et/ou le zinc est sous la forme de gluconate de zinc.

4. Utilisation selon l'une des revendications 1 à 3, dans laquelle ladite composition est sous la forme d'une dragée, d'une gélule, d'une suspension, d'un gel, d'une émulsion, d'une solution buvable, d'un comprimé à avaler ou à croquer, d'une capsule, d'un granulé à dissoudre, d'un sirop, d'une tablette ou d'une ampoule buvable.

5. Utilisation selon l'une des revendications 1 à 4, dans laquelle ledit au moins un acide gras polyinsaturé est présent en une teneur comprise entre 1% et 80% en poids par rapport au poids total de la composition cosmétique.

6. Utilisation selon l'une des revendications 1 à 5, **caractérisée en ce que** la vitamine D est présente en une teneur comprise entre 0,00001% et 0,15% en poids par rapport au poids total de la composition cosmétique.

7. Composition cosmétique destinée à une administration par voie orale consistant en :
- au moins un acide gras polyinsaturé, la teneur totale en acide gras polyinsaturé étant comprise entre 1% et 80 %,
- de la vitamine D en une teneur comprise entre 0,00001% et 0,15%,
- un caroténoïde, ledit caroténoïde étant du lycopène, et la teneur totale en caroténoïde étant comprise entre 0,01% et 6%,
- du zinc en une teneur comprise entre 0,001% et 30%,
- de la vitamine E en une teneur comprise entre 0,001% et 10%,
- de la vitamine C en une teneur comprise entre 0,001% et 30%, et
- optionnellement, au moins un excipient,
les pourcentages étant donnés en poids par rapport au poids total de la composition cosmétique, et
**caractérisée en ce que** ledit acide gras polyinsaturé est mis en œuvre sous la forme d'une huile de poisson et d'une huile de pépins de cassis et **en ce que** ladite composition ne comprend pas de vitamine B.

8. Composition cosmétique selon la revendication 7, **caractérisée en ce que** le lycopène est mis en œuvre sous la forme d'un extrait de tomate et/ou le zinc est sous la forme de gluconate de zinc.

9. Procédé cosmétique pour améliorer la brillance de la chevelure et/ou améliorer la croissance de fibres capillaires épaisses et/ou améliorer la douceur et la vigueur des fibres capillaires, comprenant l'administration par voie orale à un individu d'une composition cosmétique telle que définie dans l'une des revendications 1 à 8.

## Patentansprüche

1. Kosmetische Anwendung, auf oralem Wege, einer kosmetischen Zusammensetzung, bestehend aus mindestens einer mehrfach ungesättigten Fettsäure, die in Form eines Fischöls und eines Kernöls von schwarzen Johannisbeeren ausgeführt ist, Vitamin D, einem Karotinoid, wobei das Karotinoid Lycopin ist, Zink, Vitamin E, Vitamin C und wahlweise mindestens einem Trägerstoff, zum Verbessern des Glanzes des Haars und/oder Verbessern des Wachstums von dicken Haarfasern und/oder Verbessern von Weichheit und Festigkeit der Haarfasern, wobei die kosmetische Zusammensetzung kein Vitamin B aufweist.

2. Anwendung gemäß Anspruch 1, **dadurch gekennzeichnet, dass** das Vitamin D ausgewählt ist aus Vitamin D3 und Vitamin D2, vorzugsweise Vitamin D3.

3. Anwendung gemäß Anspruch 1 oder 2, **dadurch gekennzeichnet, dass** das Lycopin in der Form eines Tomatenextrakts ausgeführt ist und/oder das Zink in der Form von Zink-Gluconat vorliegt.

4. Anwendung gemäß einem der Ansprüche 1 bis 3, wobei die Zusammensetzung in der Form eines Dragees, einer Kapsel, einer Suspension, eines Gels, einer Emulsion, einer trinkbaren Lösung, einer zu schluckenden oder zu kauenden Filmtablette, einer Kapsel, eines aufzulösenden Granulats, eines Sirups, einer Tablette oder einer trinkbaren Ampulle vorliegt.

5. Anwendung gemäß einem der Ansprüche 1 bis 4, wobei die mindestens eine mehrfach ungesättigte Fettsäure in einem Gehalt von zwischen einschließlich 1 Gewichts-% und 80 Gewichts-% bezogen auf das Gesamtgewicht der kosmetischen Zusammensetzung vorliegt.

6. Anwendung gemäß einem der Ansprüche 1 bis 5, **dadurch gekennzeichnet, dass** das Vitamin D in einem Gehalt von zwischen einschließlich 0,00001 Gewichts-% und 0,15 Gewichts-% bezogen auf das Gesamtgewicht der kosmetischen Zusammensetzung vorliegt.

7. Kosmetische Zusammensetzung zur oralen Verabreichung, bestehend aus:
- mindestens einer mehrfach ungesättigten Fettsäure, wobei der Gesamtgehalt an mehrfach ungesättigter Fettsäure zwischen einschließlich 1% und 80% ist,
- Vitamin D in einem Gehalt von zwischen einschließlich 0,00001% und 0,15%,
- einem Karotinoid, wobei das Karotinoid Lycopin ist und der Gesamtgehalt an Karotinoid zwischen einschließlich 0,01% und 6% ist,
- Zink in einem Gehalt zwischen einschließlich 0,001% und 30%,
- Vitamin E in einem Gehalt von zwischen einschließlich 0,001% und 10%,
- Vitamin C in einem Gehalt von zwischen einschließlich 0,001% und 30%, und
- wahlweise mindestens einem Trägerstoff,
wobei die Prozentangaben als Gewicht bezogen auf das Gesamtgewicht der kosmetischen Zusammensetzung angegeben sind, und
**dadurch gekennzeichnet, dass** die mehrfach ungesättigte Fettsäure in Form eines Fischöls und eines Kernöls von schwarzen Johannisbeeren ausgeführt ist und die Zusammensetzung kein Vitamin B aufweist.

8. Zusammensetzung gemäß Anspruch 7, **dadurch gekennzeichnet, dass** das Lycopin in der Form eines Tomatenextrakts ausgeführt ist und/oder das Zink in der Form von Zink-Gluconat vorliegt.

9. Kosmetische Behandlung zum Verbessern des Glanzes des Haars und/oder Verbessern des Wachstums von dicken Haarfasern und/oder Verbessern von Weichheit und Festigkeit der Haarfasern, aufweisend die orale Verabreichung einer kosmetischen Zusammensetzung, wie sie in einem der Ansprüche 1 bis 8 definiert ist, an eine Person.

## Claims

1. A cosmetic use, via an oral route, of a cosmetic composition consisting of at least one polyunsaturated fatty acid applied in the form of a fish oil and a blackcurrant pip oil, vitamin D, a carotenoid, said carotenoid being lycopene, zinc, vitamin E, vitamin C and, optionally, at least one excipient, for improving the gloss of the hair and/or improving the growth of thick capillary fibers and/or improving the gentleness and strength of the capillary fibers, wherein said cosmetic composition does not comprise any vitamin B.

2. The use according to claim 1, **characterized in that** vitamin D is selected from among vitamin D3 and vitamin D2, preferably vitamin D3.

3. The use according to claim 1 or 2, **characterized in that** lycopene is applied in the form of a tomato extract and/or zinc is in the form of zinc gluconate.

4. The use according to any one of claims 1 to 3, wherein said composition is in the form of a dragee, a gelatin capsule, a suspension, a gel, an emulsion, a drinkable solution, a tablet to be swallowed or to be crunched, a capsule, a granule to be dissolved, a syrup, a tablet or a drinkable ampoule.

5. The use according to any one of claims 1 to 4, wherein said at least one polyunsaturated fatty acid is present in a content comprised between 1% and 80% by weight based on the total weight of the cosmetic composition.

6. The use according to any one of claims 1 to 5, **characterized in that** the vitamin D is present in a content comprised between 0.00001% and 0.15% by weight based on the total weight of the cosmetic composition.

7. A cosmetic composition intended for oral administration consisting of:
- at least one polyunsaturated fatty acid, the total content of polyunsaturated fatty acid being comprised between 1% and 80%,
- vitamin D in a content comprised between 0.00001% and 0.15%,
- a carotenoid, said carotenoid being lycopene, and the total carotenoid content being comprised between 0.01% and 6%,
- zinc in a content comprised between 0.001% and 30%,
- vitamin E in a content comprised between 0.001% and 10%,
- vitamin C in a content comprised between 0.001% and 30%, and
- optionally, at least one excipient,
the percentages being given by weight based on the total weight of the cosmetic composition, and
**characterized in that** said polyunsaturated fatty acid is applied in the form of a fish oil and a blackcurrant pip oil and **in that** said composition does not comprise any vitamin B.

8. The cosmetic composition according to claim 7, **characterized in that** lycopene is applied in the form of a tomato extract and/or zinc is in the form of zinc gluconate.

9. A cosmetic method for improving the gloss of the hair and/or improving the growth of thick capillary fibers and/or improving the gentleness and strength of the capillary fibers, comprising the oral administration to an individual of a cosmetic composition as defined in any one of claims 1 to 8.
